# EUROPÄISCHE PATENTANMELDUNG

(11) **EP 4 407 306 A1**
(43) Veröffentlichungstag der Anmeldung: **31.07.2024**
(21) Anmeldenummer: 24153684.6
(22) Anmeldetag: 24.01.2024
(51) Int. Cl.: G01N 27/08

(54) **ELEKTRODE FÜR DIALYSEGERÄTE**

(30) Priorität: 26.01.2023 DE 102023101890
(71) Anmelder: B. Braun Avitum AG, 34212 Melsungen (DE)
(72) Erfinder: BERTRAM, Rolf, 34286 Bergheim (DE)
(74) Vertreter: Winter, Brandl - Partnerschaft mbB

(57) **Zusammenfassung**

Offenbart ist eine Elektrode (16) mit einem Hauptkörper (22), der eine Durchgangsausnehmung (18) hat, die zwei zueinander parallele Stirnseiten (30) des Hauptkörpers (22) miteinander verbindet. Die Durchgangsausnehmung (18) weist einen elektrisch leitfähigen Innenmantel (20) auf, der von einer zu messender Dialysierflüssigkeit oder von deren Komponenten durchströmbar ist. Die Elektrode (16) hat weiterhin einen am Außenumfang des Hauptkörpers (22) angeordneten elektrischen Kontakt (2). Der Innenmantel (20) und der elektrische Kontakt (2) sind gemeinsam aus einem ersten elektrisch leitfähigen Material gebildet und der Hauptkörper (22) ist ein von dem Innenmantel (20) und dem elektrischen Kontakt (2) getrenntes Trägerbauteil aus einem zweiten vorzugsweise elektrisch nicht leitfähigen Material.

## Beschreibung

### Technisches Gebiet

Die vorliegende Offenbarung betrifft eine Elektrode, die zur Leitfähigkeitsmessung bei der Herstellung von Dialysierflüssigkeit in Dialysegeräten eingesetzt wird. Dazu werden zwei derartige Elektroden mittels eines Abstandhalters zueinander beabstandet angeordnet, so dass die Durchgangsausnehmungen der Elektroden zusammen mit dem dazwischen angeordneten Durchgangskanal des Abstandhalters einen Messkanal ausbilden, der dann während der Herstellung bzw. Aufbereitung der Dialysierflüssigkeit von deren Komponenten Permeat und Bikarbonat-Konzentrat oder danach von der fertigen Dialysierflüssigkeit durchströmt werden bzw. wird.

### Stand der Technik

Im Internet unter www.schunk-group.com/kohlenstofftechnik/de/produkte/detail/dialysemesszellen~p18593 ist eine als Dialysemesszelle bezeichnete Elektrode offenbart, deren Hauptkörper einstückig aus Kohlenstoff bzw. Graphit gefertigt ist. In diesem Hauptkörper ist eine Durchgangsausnehmung gebildet, die von der Dialysierflüssigkeit oder deren Komponenten Permeat und Bikarbonat-Konzentrat durchströmt wird, deren Leitfähigkeit bestimmt werden soll.

Die Herstellung der Hauptkörper derartiger Elektroden aus massivem Graphit ist kostenintensiv, da der Graphit vorher gepresst und dann der Hauptkörper aus dem Vollen gefertigt werden muss. Weiterhin ist es schwierig, an derartigen Hauptkörpern den elektrischen Kontakt in Form der Litze zu befestigen.

### Kurzbeschreibung der Offenbarung

Es ist die Aufgabe der vorliegenden Offenbarung, eine Elektrode zu schaffen, bei der die vorstehenden Nachteile vermieden sind.

Diese Aufgabe wird durch eine Elektrode bzw. eine Dialysierflüssigkeits-Leitfähigkeitselektrode mit den Merkmalen des Patentanspruchs 1 gelöst.

Die Elektrode bzw. Dialyse-Leitfähigkeitsmesselektrode gemäß der Offenbarung hat einen (Puck-artigen) Hauptkörper, der eine (axial sich erstreckende) Durchgangsausnehmung oder Kanal hat oder umgibt, die zwei (zueinander parallele bzw. parallel-beabstandete) Stirnseiten des Hauptkörpers miteinander verbindet. In der Durchgangsausnehmung ist ein elektrisch leitfähiger Innenmantel angeordnet, der von einer zu messender Dialysierflüssigkeit oder von deren Komponenten durchströmbar ist. In anderen Worten ausgedrückt ist ein den Innenmantel definierendes Innenrohr aus einem elektrisch leitfähigen Material vorgesehen, das von einem Block aus einem vorzugsweise elektrisch nichtleitenden zweiten Material umgeben ist. Der Materialblock bildet dabei einen Hauptkörper der Elektrode. Das Innenrohr ist entweder als ein zum Materialblock separat gebildetes Bauteil oder durch Beschichten der im Materialblock vorgesehenen Durchgangsausnehmung ausgebildet.

An der Außenseite, insbesondere am Außenumfang des Hauptkörpers ist ein elektrischer Kontakt angeordnet, der sich vorzugsweise radial von der Durchgangsausnehmung oder dem Innenmantel weg erstreckt und mit dem Innenmantel elektrisch verbunden ist. Der Innenmantel und der elektrische Kontakt sind vorzugsweise gemeinsam aus einem ersten elektrisch leitfähigen Material gebildet. Der Hauptkörper ist vorzugsweise ein von dem Innenmantel und dem elektrischen Kontakt getrenntes Trägerbauteil, das aus einem zweiten, vorzugsweise elektrisch nichtleitfähigen Material (weiter vorzugsweise aus Vollmaterial) gefertigt ist. Es ist auch möglich, den Hauptkörper aus elektrisch leitfähigem Material zu fertigen.

In vorteilhafter Weise besteht die Elektrode also aus einer elektrisch leitfähigen Hülse (Innenrohr/Innenmantel) mit einer daran angeformten oder fixierten, elektrisch leitfähigen Fahne oder Litze, die gemeinsam von einem elektrisch isolierenden Materialblock/Trägerbauteil ummantelt/umgeben sind, derart, dass die Hülse innenwandig zumindest teilweise frei exponiert (nicht vom Materialblock abgedeckt) verbleibt sowie deren axiale Stirnseiten zumindest teilweise nach außen offen sind (und damit die Hülse durchströmbar ist) und die Fahne oder Litze zumindest an deren freiem Endabschnitt eine (von außen frei zugängliche) elektrische Anschlussstelle bildet.

Die Herstellungskosten der offenbarungsgemäßen Elektrode sind gegenüber dem Stand der Technik vermindert, da das zweite (elektrisch isolierende) Material des Trägerbauteils beispielsweise ein Kunststoff, eine Keramik oder dergleichen Isolationsmaterial sein kann und daher weniger teuer als das Graphit des Standes der Technik ist und einfach, beispielsweise durch Spritzgussformen hergestellt werden kann. Weiterhin ist die Fertigung der elektrisch leitfähigen Bestandteile der Elektrode vereinfacht da diese im Wesentlichen nur aus Innenmantel und Fahne / Litze bestehen. Es bieten sich mit dem Hauptkörper gemäß der Offenbarung auch vereinfachte Möglichkeiten zusätzliche Dichtringe an den beiden axialbeabstandeten Stirnseiten des Hauptkörpers/Materialblocks/Trägerbauteils zu befestigen, wodurch die Anzahl der Einzelteile der übergeordneten Leitfähigkeitssonde vermindert ist. So ist es beispielsweise möglich, zwei Dichtringe über axiale Stege vorzugsweise stoffeinstückig in einem vorbestimmten Axialabstand zueinander zu verbinden und anschließend vom Materialblock beispielsweise durch Spritzgießen zu umschließen. Auf diese Weise werden die Dichtringe fest und sicher am Materialblock (durch mechanische Kopplung und nicht nur per Klebwirkung) gehalten.

Damit ist der Zusammenbau der Elektrode gemäß der Offenbarung vereinfacht.

Der Hauptkörper und der in der Durchgangsausnehmung angeordnete oder gebildete Innenmantel können in per se aus dem Stand der Technik bekannter Weise kreiszylindrisch und konzentrisch zueinander sein. Es ist aber auch möglich, dass der Hauptkörper (und ggf. der Innenmantel) eine hierzu abweichende Form hat, z.B. quaderförmig ist. Dabei kann der Querschnitt des Hauptkörpers (quer zur Durchflussrichtung oder Mittelachse des Innenmantels) quadratisch sein. Dies kann einer vereinfachten Montage und/oder Lagepositionierung dienen, wenn zwei derartige Elektroden über zumindest einen Abstandhalter zueinander beabstandet montiert werden. Ggf. kann die Querschnittsform des Innenmantels von der Querschnittsform des Hauptkörpers abweichen.

Gemäß dem Stand der Technik ist der elektrische Kontakt eine Litze, die als Stampfkontakt ausgebildet bzw. befestigt ist. Dem gegenüber ist es offenbarungsgemäß bevorzugt, wenn der elektrische Kontakt (Fahne) ein Flachkontakt und/oder ein Steckkontakt ist. Damit ist die Herstellung des elektrischen Kontakts vereinfacht. Der elektrische Kontakt kann auch ein Durchgangsloch/-Schlitz zur leichteren Befestigung einer separaten Anschlusslitze haben, die nicht Bestandteil der Elektrode ist.

Gemäß einem ersten Dichtkonzept ist an zumindest einer der Stirnseiten der eine Dichtring angeordnet, der eine an der Stirnseite gebildete Mündung der Durchgangsausnehmung vollumfänglich umgibt, und der stoffschlüssig, also chemisch an der Stirnseite befestigt ist. Dabei wird es besonders bevorzugt, wenn an beiden Stirnseiten und an beiden Mündungen jeweils ein derartiger Dichtring angeordnet ist. Dann sind alle Dichtungen bereits mit der Fertigung des Hauptkörpers vormontiert und die Anzahl der Einzelteile der übergeordneten Leitfähigkeitssonde/Messzelle ist reduziert, wodurch deren Montage vereinfacht ist. Insbesondere ist die Gefahr ausgeschlossen, dass beim Zusammenbau der Leitfähigkeitssonde ein Dichtring vergessen oder nicht korrekt positioniert wird.

Gemäß einem zweiten Dichtkonzept kann der mindestens eine Dichtring auch formschlüssig an der Stirnseite befestigt sein.

Bei einer bevorzugten konkreten Ausgestaltung des zweiten Dichtkonzeptes ist an beiden Stirnseiten jeweils ein Dichtring angeordnet, der die jeweilige an der Stirnseite gebildete Mündung der Durchgangsausnehmung vollumfänglich umgibt. Die beiden Dichtringe bilden gemeinsam mit mindestens einem Verbindungselement/Steg ein (stoff-)einstückiges Dichtbauteil insbesondere aus Silikon.

Im Unterschied zu der vorstehenden Beschreibung ist es alternativ möglich, dass sich das mindestens eine Verbindungselement/Steg zwischen den beiden Dichtringen durch ein als Durchgangsloch im Hauptkörper gebildetes Verbindungsloch erstreckt. Das Dichtbauteil kann in diesem Fall an den bereits (vor-)gefertigten Hauptkörper in einem Schuss gespritzt werden.

So können (gleichmäßig) am Außenumfang des Innenmantels der Durchgangsausnehmung verteilt zwei, drei oder vier Verbindungslöcher vorgesehen sein, durch die sich jeweils ein Verbindungselement des Dichtbauteils erstreckt.

Der Hauptkörper ist aus dem zweiten vorzugsweise nicht elektrisch leitfähigen Material gefertigt. Dieses kann biokompatibles PPE und PS oder PEI oder PSU sein oder zumindest enthalten.

Der Innenmantel und der elektrische Kontakt (Fahne) sind gemeinsam aus dem ersten elektrisch leitfähigen Material gebildet. Dieses kann Kupfer oder Messing, Silber oder Hartgold oder Graphit sein oder zumindest enthalten. Das erste Material insbesondere in Form von Hartgold oder Graphit kann als Beschichtung auf dem Hauptkörper aufgetragen sein.

Der elektrische Kontakt kann über einen Verbindungs-Zwischenabschnitt mit dem Innenmantel verbunden und elektrisch kontaktiert sein. Der Verbindungs-Zwischenabschnitt (nachfolgend einfach als Verbindungsabschnitt bezeichnet) dient quasi als elektrische Brücke zwischen dem radial vom Innenmantel beabstandeten und zumindest teilweise außerhalb des Hauptkörpers angeordneten elektrischen Kontakt und dem Innenmantel.

Bei einem ersten Ausführungsbeispiel der Dialyse-Leitfähigkeitsmesselektrode bilden der Innenmantel und der elektrische Kontakt ein (stoff-)einstückiges Stanz-Biege-/ Stanz-Tiefzieh-Bauteil. Der Hauptkörper ist ein Kunststoff-Spritzgussteil, das um den Innenmantel und zumindest um den Verbindungsabschnitt oder teilweise um den elektrischen Kontakt (Fahne) herumgespritzt ist.

Offenbart ist auch ein erstes Ausführungsbeispiel eines Herstellungsverfahrens einer Dialyse-Leitfähigkeitsmesselektrode, bei dem zunächst das Stanz-Biege-Bauteil aus dem ersten Material gefertigt wird, und danach der Hauptkörper aus dem zweiten Material um des Stanz-Biege-Bauteil herumgespritzt wird.

Bei einer konkreten Ausgestaltung des ersten Ausführungsbeispiels weist das Stanz-Biege-Bauteil einen den Innenmantel ausbildenden Zylinder/Hülse auf, an dem ein Flansch oder Kragen gebildet ist. Der Flansch/Kragen ist an einer der Stirnseiten des Hauptkörpers angeordnet, und der Verbindungsabschnitt/Fahne erstreckt sich radial weg von dem Flansch.

Bei dünneren Flachkontakten oder Steckkontakten (zungenförmige Kontaktfahnen) kann es von großem Vorteil sein, wenn der Hauptkörper eine (stoff-)einstückig angeformte/ausgebildete Kontaktstütze ausbildet/hat, quasi in Form einer Schiene aus elektrisch nichtleitendem Material, an welcher der elektrische Kontakt in Kontaktlängsrichtung einseitig aufgelegt ist und sich so daran abstützt (und daher nichtmehr umgebogen werden kann). Wenn der elektrische Kontakt zusätzlich das Durchgangsloch für die Litze hat, dann hat selbstverständlich auch die Kontaktstütze ein entsprechendes (deckungsgleiches) Durchgangsloch für die Litze.

Bei einem zweiten Ausführungsbeispiel der Dialyse-Leitfähigkeitsmesselektrode sind der Innenmantel und der elektrische Kontakt per MID-Technik (molded interconnect device) oder mittels einer ähnlichen Beschichtungstechnik als Beschichtung auf den Hauptkörper und auf dessen Kontaktstütze aufgebracht. Vorzugsweise bestehen der aufgetragene Innenmantel und der aufgetragene elektrische Kontakt aus Graphit oder Kupfer.

Offenbart ist auch ein zweites Ausführungsbeispiel eines Herstellungsverfahrens einer Dialyse-Leitfähigkeitsmesselektrode, bei dem zunächst der Hauptkörper aus dem zweiten Material gefertigt wird, und danach der Innenmantel und der elektrische Kontakt aus dem ersten Material auf den Hauptkörper aufgetragen werden.

Bei einer Ausgestaltung des zweiten Ausführungsbeispiels ist der Verbindungsabschnitt an einer der beiden Stirnseiten des Hauptkörpers angeordnet.

Bei einem dritten Ausführungsbeispiel der Dialyse-Leitfähigkeitsmesselektrode sind der Innenmantel und der elektrische Kontakt und der Hauptköper mittels eines additiven Verfahrens (3D-Druck) gebildet.

Offenbart ist auch ein additives Herstellungsverfahren einer Dialyse-Leitfähigkeitsmesselektrode, wobei der Innenmantel und der elektrische Kontakt aus dem ersten Material und der Hauptköper aus dem zweiten Material mittels eines Verfahrens (3D-Druck) gleichzeitig bzw. in einem Vorgang gebildet werden.

Bei einer konkreten Ausgestaltung des dritten Ausführungsbeispiels ist der Verbindungsabschnitt geschützt im Innern des Hauptkörpers angeordnet.

Das elektrisch leitende erste Material, aus dem der elektrische Kontakt und der Innenmantel und ggf. auch der Zylinder und ggf. auch der Verbindungsabschnitt gefertigt sind, kann ein nicht-metallischer und elektrisch leitender (moderner) Kunststoff sein.

Für eine Leitfähigkeitsmessung müssen wenigstens zwei Elektroden vorzugsweise gemäß der vorstehenden Offenbarung in einem (vor-)bestimmten Axialabstand zueinander (in einem Leitungssystem) angeordnet werden, wofür bevorzugt ein Abstandshalter vorgesehen ist.

Der Abstandhalter gemäß der Offenbarung wird hierfür zusammen mit Dialyse-Leitfähigkeitsmesselektroden in eine Leitfähigkeitssonde beispielsweise eines Dialysegerätes insbesondere eines Hämodialysegeräts eingebaut und ist als solches dafür ausgelegt, alleinig oder in Zusammenschluss mit weiteren/zusätzlichen Abstandshaltern gleichen Aufbaus die Leitfähigkeits-Messelektroden in ein Leitungssystem vorzugsweise Dialysierflüssigkeits-Leitungssystems des Dialysegeräts zu integrieren. Der Abstandhalter weist einen Durchgangskanal mit zwei axialbeabstandeten Mündungen auf, wobei an jeder Mündung ein Anlage-/Montagebereich für eine Leitfähigkeitsmesselektrode vorzugsweise gemäß vorstehender Offenbarung oder einen weiteren Abstandshalter vorgesehen ist, wobei der jeweilige Anlage-/Montagebereich eine die jeweilige Mündung umgebende (Elektroden-)Anlagefläche aufweist. An den beiden Anlagebereichen ist jeweils eine eigene (unabhängig und separat betätigbare und funktionierende) Spannvorrichtung vorgesehen, derart, dass die beiden Spannvorrichtungen des Abstandhalters unabhängig voneinander aktivierbar und deaktivierbar sind. Bei ihrer Aktivierung sind die beiden Spannvorrichtungen des Abstandhalters unabhängig voneinander in Wirkverbindung beispielsweise mit einer jeweiligen entsprechenden Spannvorrichtung eines Anlagebereiches eines weiteren Abstandhalters oder Abschlussflansches oder einer Elektrode vorzugsweise gemäß der vorliegenden Offenbarung bringbar.

Bei einer fertigungstechnisch einfachen Ausgestaltung des Abstandhalters ist der Durchgangskanal gerade und dabei normal bzw. senkrecht zu den beiden Anlageflächen ausgerichtet.

Aus Gründen der korrekten Formulierung der Merkmale nur genau eines beanspruchten Abstandhalters ist der Begriff "Spannvorrichtung" als ein Teil von zwei Teilen zu verstehen, die zusammen für eine Einspannung einer Elektrode nötig sind.

Nach dem gleichen Prinzip ist auch eine Anordnung offenbart, die zwei Abstandhalter und dazwischen eine Dialyse-Leitfähigkeitsmesselektrode aufweist, wobei jeder Abstandhalter einen axialen Durchgangskanal und zwei quer dazu angeordnete Anlageflächen aufweist, wobei an zwei aufeinander zuweisenden Anlageflächen der beiden Abstandhalter jeweils eine Stirnseite der Dialyse-Leitfähigkeitsmesselektrode anliegt. Die Dialyseleitfähigkeitsmesselektrode hat eine Einspannung, die aus zwei Spannvorrichtungen gebildet ist, die an den beiden aufeinander zuweisenden Anlagebereichen der beiden Abstandhalter gebildet sind.

Mit dem definierten Abstandshalter und mit der definierten Anordnung ist die Dialyse-Leitfähigkeitsmesselektrode unabhängig von weiteren Einspannungen der betroffenen Leitfähigkeitssonde einspannbar bzw. eingespannt und kann, wenn z.B. ein Leck festgestellt wird, nur an der betroffenen Einspannung wieder gelöst oder geöffnet werden, z.B. um einen fehlenden/beschädigten Dichtring zwischen der Anlagefläche des Abstandhalters und der Stirnfläche der Elektrode zu ergänzen oder um seinen Sitz zu korrigieren.

Weiterhin hat die individuelle Einspannung gemäß der Offenbarung gegenüber der durchgehenden verketteten Einspannung des Standes der Technik den Vorteil, dass auf die individuelle Einspannung nur die Kräfte von zwei Dichtringen wirkt, die zusammen mit der Elektrode zwischen den aufeinander zuweisenden Anlageflächen zweier Abstandhalter eingespannt sind.

Die beiden Anlageflächen haben vorzugsweise jeweilige zumindest abschnittsweise umlaufende Außenränder, um die anliegende Elektrode zu positionieren und in Position zu halten. Die beiden Außenränder können rund oder auch eckig (z.B. quadratisch) sein. Entsprechend hat die Elektrode einen Hauptkörper mit einem runden oder eckigen (z.B. quadratischen) Querschnitt.

Vorzugsweise ist an einem der beiden Außenränder oder an beiden Außenrändern eine Ausnehmung (Kerbe) für einen elektrischen Kontakt der Elektrode gebildet. Wenn die beiden Außenränder nicht komplett umlaufend sind, können sie z.B. durch die Ausnehmung (Kerbe) unterbrochen sein.

Bei einer besonders bevorzugten Ausgestaltung ist an beiden Anlageflächen und an beiden Mündungen jeweils ein Dichtring angeordnet. Damit benötigen die Elektroden (ihrerseits) keine Dichtringe, so dass die Elektroden mit verringertem Außendurchmesser hergestellt werden können. Weiterhin können die Stirnseiten der Elektroden ohne Nut für Dichtringe und/oder flächig ausgeführt werden.

Bei einem ersten Dichtkonzept des Abstandhalters können die beiden Dichtringe stoffschlüssig, also chemisch an der Anlagefläche befestigt sein.

Bei einem zweiten Dichtkonzept können die beiden Dichtringe formschlüssig an der Anlagefläche befestigt sein.

Beide genannten Dichtkonzepte haben den Vorteil, dass durch die (insbesondere beidseitige) Befestigung der Dichtringe am Abstandhalter beim Zusammenbau der übergeordneten Leitfähigkeitssonde die Teileanzahl deutlich verringert ist, und dass Fehler wegen fehlender oder nicht korrekt eingesetzter Dichtringe ausgeschlossen sind.

Bei einer besonders bevorzugten Ausgestaltung des zweiten Dichtkonzepts ist an beiden Anlageflächen jeweils ein Dichtring angeordnet, der die jeweilige Mündung des Durchgangskanals vollumfänglich umgibt. Zur Herstellung des Formschlusses sind die beiden Dichtringe gemeinsam mit mindestens einem Verbindungselement/Steg zu einem (stoff-)einstückigen Dichtbauteil insbesondere aus Silikon gebildet. Das mindestens eine Verbindungselement erstreckt sich axial zwischen den beiden Dichtringen.

Bei einer Weiterbildung des Abstandhalters sind die beiden Anlagebereiche jeweils von einem Flansch gebildet, wobei sich zwischen den beiden Flanschen ein Hals mit gegenüber den beiden Flanschen vermindertem Durchmesser erstreckt. In dem Hals ist ein mittlerer Abschnitt des Durchgangskanals gebildet.

Bei einem Ausführungsbeispiel umfasst oder ist die Einspannung der Elektrode eine Ringschnappverbindung. Damit umfasst oder ist die beanspruchte Spannvorrichtung des Abstandhalters eine Seite dieser Ringschnappverbindung.

Die Ringschnappverbindung ist fertigungstechnisch einfach und bietet dabei maximalen Halt zwischen den beiden betroffenen Abstandhaltern, wenn sie kreis- oder kreisbogenförmig ausgestaltet ist und sich vollumfänglich oder weitgehend oder abschnittsweise an den beiden aufeinander zuweisenden Außenrändern erstreckt.

Vorzugsweise weist die Ringschnappverbindung einen umlaufenden äußeren Wulst an dem einen Außenrand und eine umlaufende innere Nut an dem anderen Außenrand auf.

Die Geometrie der Ringschnappverbindung könnte alternativ auch eine lösbare oder nicht lösbare Rasthakengeometrie sein.

XSBei einem weiteren Ausführungsbeispiel umfasst oder ist die Einspannung der Elektrode eine Clipsverbindung. Damit umfasst oder ist die beanspruchte Spannvorrichtung des Abstandhalters eine Seite dieser Clipsverbindung. Vorzugsweise ist die Clipsverbindung mehrteilig und am Umfang verteilt.

Die Einspannung kann auch eine Bügelverbindung sein, die mindestens einen Bügel umfasst. Der Bügel hat zwei Schenkel, von den einer in ein Bügelloch des Anlagebereiches des einen Abstandhalters eingesetzt ist, während der andere Schenkel in ein entsprechendes Bügelloch des benachbarten Anlagebereiches des anderen Abstandhalters eingesetzt ist.

Gemäß einem bevorzugten Ausführungsbeispiel wird mit den beiden aufeinander zuweisenden Spannvorrichtungen der beiden Abstandhalter eine Bügelverbindung geschaffen, die zwei Bügel und vier Bügellöcher hat, wobei die beiden Bügel auf einander gegenüberliegenden Seiten des Durchgangskanals angeordnet sind.

Die drei genannten Einspannungen können auch beliebig kombiniert werden. So kann die Bügelverbindung mit der Ringschnappverbindung oder mit der Clipsverbindung kombiniert werden.

Die Leitfähigkeitssonde gemäß der Offenbarung hat mindestens einen, (vorzugsweise mehrere) vorbeschriebenen Abstandhalter und mindestens zwei (vorzugsweise mehr als zwei) Dialyse-Leitfähigkeitsmesselektroden, wobei die Anzahl der Dialyse-Leitfähigkeitsmesselektroden um eins größer ist, als die Anzahl der Abstandhalter. An den beiden äußersten Dialyse-Leitfähigkeitsmesselektroden eines Messkanals der Leitfähigkeitssonde ist jeweils innenseitig der genau eine Abstandhalter oder ein äußerster Abstandhalter angeordnet während außenseitig ein Abschlussflansch der Leitfähigkeitssonde angeordnet ist. Auch die beiden Abschlussflansche weisen jeweils einen Anlagebereich mit einer Anlagefläche auf, so dass die beiden äußersten Dialyse-Leitfähigkeitsmesselektroden jeweils zwischen den beiden aufeinander zuweisenden Anlageflächen des genau einen oder des äußersten Abstandhalters und des Abschlussflansches dichtend eingespannt sind. Auch an den Anlagebereichen der beiden Abschlussflansche ist jeweils eine Spannvorrichtung vorgesehen. Auch diese Spannvorrichtungen sind unabhängig voneinander aktivierbar und deaktivierbar, und bei ihrer Aktivierung unabhängig voneinander in Wirkverbindung mit der benachbarten Spannvorrichtung des Anlagebereiches des genau einen oder des äußersten Abstandhalters bringbar. Vorzugsweise entsprechen die beiden Spannvorrichtungen der beiden Abschlussflansche denjenigen des vorbeschriebenen Abstandhalters.

Damit ist jede einzelne Dialyse-Leitfähigkeitsmesselektrode unabhängig von weiteren Dialyse-Leitfähigkeitsmesselektoden in die Leitfähigkeitssonde eingespannt. Die Leitfähigkeitssonde kann, wenn z.B. ein Leck festgestellt wird, nur an der betroffenen Einspannung wieder gelöst oder geöffnet werden, z.B. um einen fehlenden Dichtring zwischen der Anlagefläche des Abstandhalters und der Stirnfläche der Dialyse-Leitfähigkeitsmesselektrode zu ergänzen oder dessen Sitz zu korrigieren.

Am Außenumfang des mindestens einen Abstandhalters und der mindestens zwei Dialyse-Leitfähigkeitsmesselektroden kann eine Überwurfhülse angeordnet sein. Diese kann aus Aluminium oder Edelstahlblech oder Stahlblech oder Kunststoff oder glasfaserverstärktem Kunststoff gefertigt sein. Die Überwurfhülse kann als Montagehilfe und/oder als Stützhülse dienen.

Die Überwurfhülse kann (z.B. über ihre volle Länge) einseitig offen sein, um dort Raum für die elektrischen Kontakte der Dialyse-Leitfähigkeitsmesselektroden zu lassen.

An zumindest einem der beiden Abschlussflansche kann eine Schlauchtülle und/oder eine Sensoraufnahme und/oder ein Befestigungsmittel zur Befestigung der Leitfähigkeitssonde an dem Rahmen des Dialysegerätes gebildet sein. In die Sensoraufnahme kann ein Sensor für Temperatur, Druck oder Flussgeschwindigkeit eingesetzt werden.

Alternativ oder in Ergänzung kann das Befestigungsmittel auch an der Überwurfhülse gebildet oder angeordnet sein.

### Kurzbeschreibung der Figuren

Fig. 1 zeigt eine Leitfähigkeitssonde in einer Ansicht;
Fig. 2 zeigt eine Elektrode gemäß einem ersten Ausführungsbeispiel der vorliegenden Offenbarung;
Fig. 3 zeigt eine Explosionszeichnung der Elektrode aus Fig. 2;
Fig. 4 zeigt eine Elektrode gemäß einem zweiten Ausführungsbeispiel der vorliegenden Offenbarung in einem unfertigen Zustand;
Fig. 5 zeigt der Elektrode aus Fig. 4 im fertigen Zustand;
Fig. 6 zeigt eine Elektrode gemäß einem dritten Ausführungsbeispiel der vorliegenden Offenbarung in einem unfertigen Zustand;
Fig. 7 zeigt die Elektrode aus Fig. 6 im fertigen Zustand;
Fig. 8 zeigt die Elektrode aus Fig. 7 in einer geschnittenen Darstellung;
Fig. 9 zeigt eine lange und eine kurze Version eines ersten Beispiels eines Abstandhalters mit der Elektrode aus Figs. 2 und 3 in einer Explosionszeichnung;
Fig. 10 zeigt die Anordnung aus Fig. 9 im zusammengesetzten Zustand mit zwei weiteren Elektroden;
Fig. 11 zeigt eine lange und eine kurze Version eines zweiten Beispiels eines Abstandhalters;
Fig. 12 zeigt einen oberen und einen unteren Abschnitt der Leitfähigkeitssonde aus Fig. 1 mit den Abstandhaltern aus Fig. 11 und mit mehreren Elektroden in einer geschnittenen Darstellung.

### Beschreibung der Ausführungsbeispiele

Nachstehend werden mehrere Ausführungsbeispiele der vorliegenden Offenbarung auf der Basis der zugehörigen Figuren beschrieben.

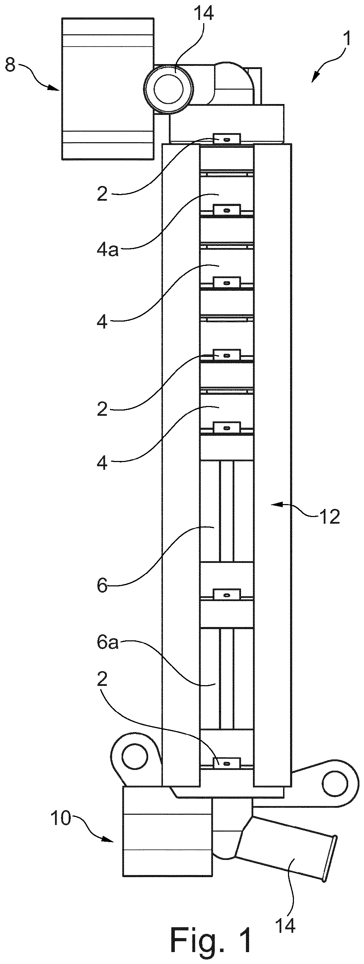

Fig. 1 zeigt eine Leitfähigkeitssonde 1. Sie hat mehrere Elektroden, die als Elektroden dienen, und von denen in Fig. 1 jeweils lediglich ein elektrischer Kontakt 2 zu erkennen ist. Die Leitfähigkeitssonde 1 wird direkt oder mittels Montageadapter (Hilfselement; Blech; spritzgegossener Träger) an einem Rahmen eines Dialysegerätes befestigt und (von einem Permeat und Bikarbonat-Konzentrat oder) von einer fertigen Dialysierflüssigkeit durchströmt.

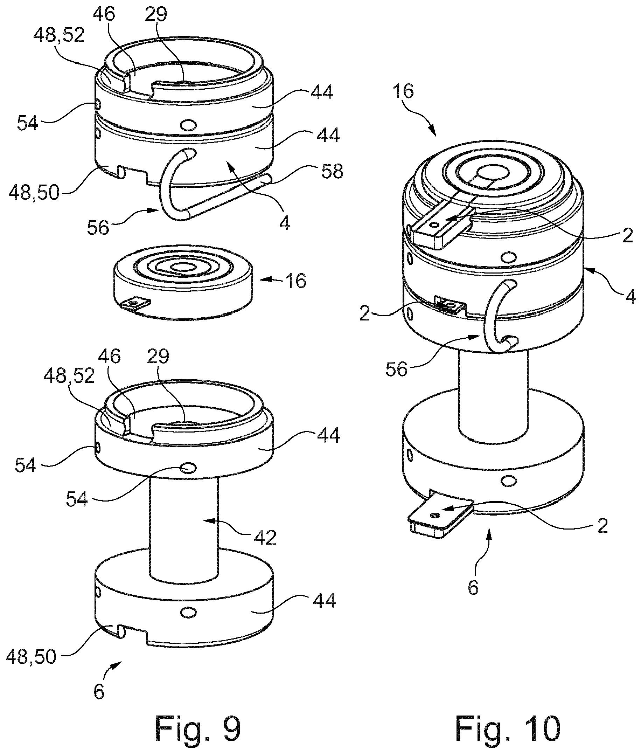

Die Elektroden werden entlang eines inneren Messkanals mit Abstandhaltern 4, 4a, 6, 6a voneinander in einem vorbestimmten Maß beabstandet. Dazu sind kürzere Abstandhalter 4, 4a und längere Abstandhalter 6, 6a vorgesehen. Mehrere Elektroden und mehrere Abstandhalter 4, 4a, 6, 6a sind abwechselnd angeordnet und mit zwei Abschlussflanschen 8, 10 verspannt. Genauer gesagt bildet der (in Fig. 1 obere) Abschlussflansch 8 direkt mit dem (in Fig. 1 darunter angeordneten) benachbarten äußersten Abstandhalter 4a eine Einspannung für die dortige äußerste Elektrode. Auf gleiche Weise bildet der (in Fig. 1 untere) Abschlussflansch 10 direkt mit dem (in Fig. 1 darüber angeordneten) benachbarten äußersten Abstandhalter 6a eine Einspannung für die dortige äußerste Elektrode. Auch die anderen Elektroden sind direkt durch die beiden benachbarten Abstandhalter 4, 4a, 6, 6a eingespannt. Die individuelle Einspannung jeder Elektrode wird mit Bezug zu den Figs. 9 bis 12 genauer erläutert.

Zwischen den beiden Abschlussflanschen 8,10 ergibt sich somit ein kreiszylindrischer Aufbau aus den Elektroden und den Abstandhalter 4, 4a, 6, 6a, der beim gezeigten Ausführungsbespiel von einer metallischen Überwurfhülse 12 umgriffen wird, die auch entfallen kann. Diese ist einseitig offen, so dass die elektrischen Kontakte 2 herausstehen können um mit einer übergeordneten Steuereinheit des Dialysegerätes elektrisch verbunden zu werden. Die Überwurfhülse 12 stelle eine Montagehilfe und/oder Abstützung dar. Weiterhin kann sie in Verbindung mit einem Zusatzteil (Adapterblech oder im Spritzgussverfahren hergestellter Adapter) eine Befestigungsmöglichkeit darstellen.

Bei der gezeigten Leitfähigkeitssonde hingegen sind an dem (in Figur 1 unteren) Abschlussflansch 10 sind Befestigungsösen zur Befestigung der Leitfähigkeitssonde 1 an einem Geräterahmen oder Hydraulikkomponenten des (nicht gezeigten) Dialysegerätes einstückig gebildet.

An beiden Abschlussflanschen 8, 10 sind jeweilige Schlauchtüllen 14 einstückig gebildet. Eine Schlauchtülle dient als Zulauf und die andere Schlauchtülle 14 als Ablauf (für Permeat und Bikarbonat-Konzentrat oder) für die fertige Dialysierflüssigkeit.

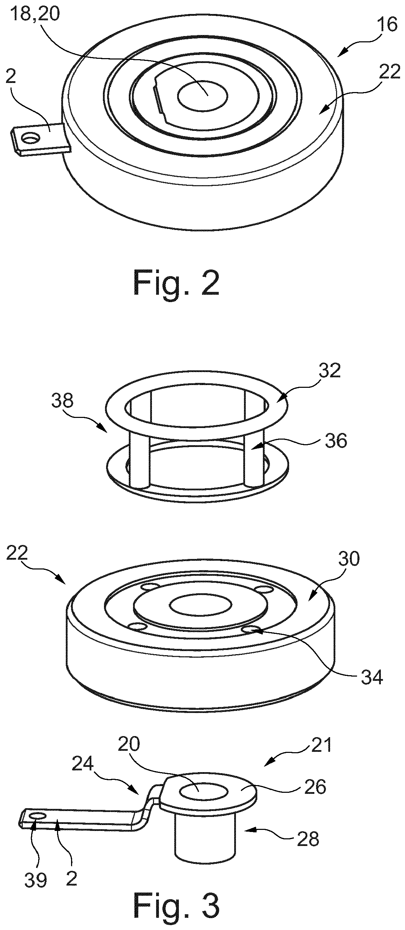

Figur 2 zeigt ein erstes Beispiel einer Elektrode 16. Sie hat eine Durchgangsausnehmung 18 mit einem elektrisch leitfähigen Innenmantel 20, der von der zu messenden Dialysierflüssigkeit oder von deren Komponenten durchströmbar ist. Die Elektrode 16 hat weiterhin einen Hauptkörper 22, an dessen Außenumfang der elektrische Kontakt 2 angeordnet ist. Der Innenmantel 20 und der elektrische Kontakt 2 sind gemeinsam aus einem ersten Material gebildet, das elektrisch leitend ist.

Figur 3 zeigt die drei wesentlichen Komponenten, aus denen die Elektrode 16 aus Fig. 2 zusammengesetzt ist. Eine Komponente ist ein Stanz-Biege-Bauteil 21, das einstückig aus dem elektrischen Kontakt 2, einem etwa s- oder stufenförmigen Verbindungsabschnitt 24, und einem Flansch 26 und einem Zylinder 28 zusammengesetzt ist. Die drei Abschnitte 2, 24, 26, 28 sind in dieser Reihenfolge aneinander befestigt, wobei der Flansch 26 an einem Endabschnitt des Zylinders 28 angeordnet ist. Damit wird eine der beiden Mündungen der Durchgangsausnehmung 18 gebildet. Der Flansch 26 ist bündig in eine der beiden (in Fig. 3 unteren) Stirnseiten 30 des Hauptkörpers 22 eingepasst. In dem Zylinder 28 ist der Innenmantel 20 gebildet, der mit dem Permeat und Bikarbonat-Konzentrat oder mit der fertige Dialysierflüssigkeit in Kontakt kommt.

Das elektrisch leitende Stanz-Biege-Bauteil 21 ist von dem Hauptkörper 22 umgeben, wobei der elektrische Kontakt 2 aus dem Hauptkörper 22 herausragt. Der Hauptkörper 22 ist bei der Elektrode 16 aus den Figs. 1 und 2 als Kunststoff-Spritzguss-Teil ausgeführt, das um das Stanz-Biege-Bauteil 21 herum gespritzt wurde.

An jeder Stirnseite 30 des Hauptkörpers 22 ist eine umlaufende Nut für einen jeweiligen Dichtring 32 gebildet, wobei die beiden Nuten durch vier gleichmäßig am Umfang verteilte Verbindungslöcher 34 miteinander verbunden sind.

In einem letzten Fertigungsschritt der Elektrode 16 werden die beiden Dichtringe 32 aus Silikon oder einem anderen Dichtwerkstoff in einem Schuss an den Hauptkörper 22 angespritzt. Dabei dingt das Silikon auch durch die vier Verbindungslöcher 34 und verbindet so die beiden Dichtringe 32. Es entsteht das in Figur 3 oben gezeigte einstückige Dichtbauteil 38. Damit sind die beiden Dichtringe 32 formschlüssig an den jeweiligen Stirnseiten 30 des Hauptkörpers 22 befestigt.

Der Hauptkörper 22 und das Dichtbauteil 38 sind im Mehrkomponenten-Spritzgussverfahren hergestellt. Das zweite z.B. nicht leitende Material des Hauptkörpers 22 und das Silikon oder der andere Dichtwerkstoff des Dichtbauteils 38 sind biokompatibel.

Das Stanz-Biege-Bauteil 21 ist z.B. aus Kupfer oder Messing oder zusätzlich komplett oder partiell leitfähig biokompatibel beschichtet, z.B. mit Hartgold oder Graphit. Dies dient der Optimierung der Detektierung bzw. Signalleitung.

Der gezeigte elektrische Kontakt 2 ist ein Flachkontakt oder Steckkontakt. Der elektrische Kontakt 2 weist auch ein als Durchkontaktierung für eine Litze dienendes Durchgangsloch 39 aufweisen.

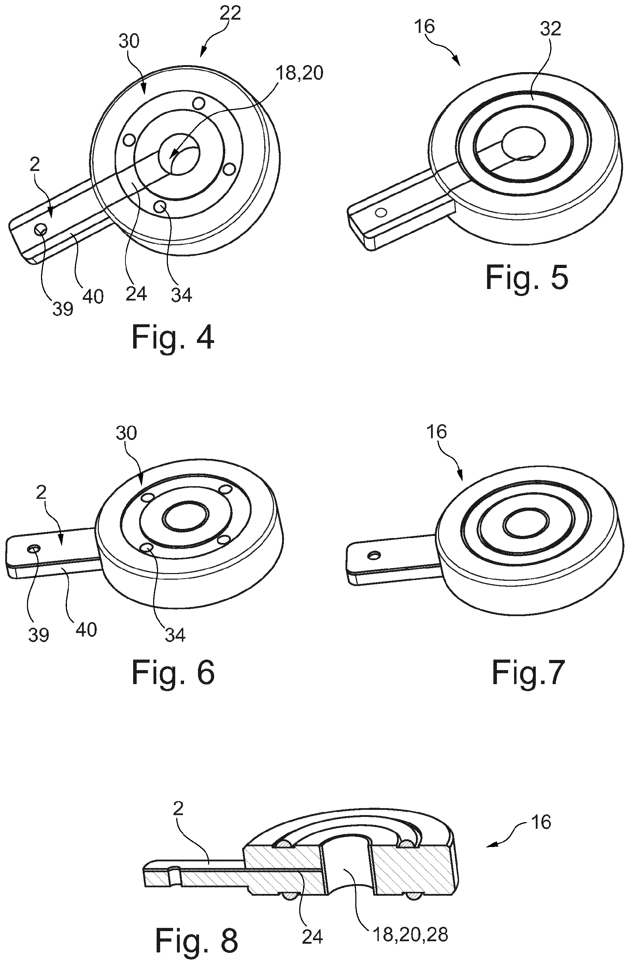

Figs. 4 und 5 zeigen ein weiteres Beispiel einer Elektrode 16, wobei Fig. 4 einen Zwischenstand der Fertigung zeigt, während die Ansicht aus Fig. 5 die fertige Elektrode 16 zeigt. Es handelt sich um eine mit einem MID-Verfahren (moulded interconnect device) oder einer ähnlichen Beschichtungstechnik hergestellte Elektrode 16. Dabei wird zunächst der Hauptkörper 22 mit einer Kontaktstütze 40 einstückig aus dem zweiten z.B. nicht leitfähigen Material (z.B. PPE+PS oder PEI oder PSU) biokompatibel gefertigt, wobei sich die Kontaktstütze 40 radial weg vom Hauptkörper 22 erstreckt.

Danach wird der elektrische Kontakt 2 mit dem Verbindungsabschnitt 24 und mit dem Innenmantel 20 aus dem leitfähigen Material, z.B. Graphit, oder Edelstahl oder Gold aufgetragen. Genauer gesagt wird der elektrische Kontakt 2 auf der Kontaktstützte 40 und der Verbindungsabschnitt 24 auf einer der Stirnseite 30 des Hauptabschnitts 22 und der Innenmantel 20 in die Durchgangsausnehmung 18 des Hauptkörpers 22 aufgetragen.

Die elektrische Kontakt 2 ist schmaler als die Kontaktstütze 40. Der Verbindungsabschnitt 24 folgt der Form der Stirnseite 30, also auch der Form der Nut für den Dichtring 32. Es ist dargestellt, dass der elektrische Kontakt 2 mit dem Verbindungsabschnitt 24 gemeinsam die Form eines durchgehenden Streifens haben.

Weiterhin abweichend von der Elektrode 16 aus Fig. 3 ist für die Ausbildung des Innenmantels 20 kein stabiler Zylinder vorgesehen. Vielmehr haben der Innenmantel 20 und der Verbindungsabschnitt 24 und der elektrische Kontakt 2 eine geringe und vorzugsweise gleiche Dicke.

Der elektrische Kontakt 2 kann als Lötpad dienen und/oder es wir das gezeigte Durchgangsloch 39 zur Befestigung einer Litze genutzt.

Figs. 6 bis 8 zeigen ein weiteres Beispiel einer Elektrode 16, wobei Fig. 6 einen Zwischenstand der Fertigung zeigt, während die Ansicht aus Fig. 7 und die geschnittene Darstellung aus Fig. 8 die fertige Elektrode 16 zeigen. In diesem Fall wurde mittels eines additiven Verfahrens (3D-Druck) die Kombination aus dem elektrisch leitfähigen Bauteil (elektrischer Kontakt 2, Verbindungsabschnitt 24 und Zylinder 28) zusammen mit dem Hauptkörper 22 aus Kunststoff zeitgleich bzw. zusammen aufgebaut (gedruckt). Danach wurde in der mit Bezug zu Figur 3 beschriebenen Weise das Dichtbauteil 38 in einem Schuss gespritzt.

Mit Blick nur auf Figs. 7 und 8 ist auch ein anderes Herstellungsverfahren für die Elektrode 16 nachvollziehbar: demnach können auch alle wesentlichen Komponenten, also der Hauptkörper 22 mit dem elektrisch leitfähigen Bauteil und mit zwei einzelnen Dichtringen 32 oder mit dem Dichtbauteil 38 mittels eines additiven Verfahrens (3D-Druck) zeitgleich bzw. in einem Vorgang aufgebaut (gedruckt) werden.

Fig. 8 zeigt, dass der elektrische Kontakt 2 und der Verbindungsabschnitt 24 in Form einer streifen- oder zungenförmigen durchgehenden Steges ausgebildet sind, der sich von einer (in Längsrichtung des Zylinders 28 betrachtet) mittleren Stelle von dem Zylinder 28 weg erstreckt.

Bei allen Elektroden 16 der Figs. 2 bis 8 können statt des in einem Schuss angespritzte Dichtbauteils 38 auch zwei einzelne Dichtringe 32 (ohne Verbindungselement 36) vorgesehen sein, die in den umlaufenden Nuten der Stirnseiten 30 angeordnet sind.

Fig. 9 zeigt eine lange und eine kurze Version eines ersten Beispiels eines Abstandhalters 4, 6 aus Fig. 1 mit der Elektrode 16 aus Figs. 2 und 3 in einer Explosionszeichnung.

Jeder Abstandhalter 4, 6 hat einen in einem Hals 42 gebildeten Durchgangskanal. Beim kürzeren Abstandhalter 4 ist der Hals 42 so kurz, dass nur eine umlaufende Nut zu erkennen ist. An jedem Endabschnitt des Halses 42 ist ein als Flansch 44 ausgestalteter Anlagebereich vorgesehen. An den Flanschen 44 ist eine Anlagefläche 46 für eine Stirnseite 30 einer Elektrode 16 gebildet.

Die Anlageflächen 46 haben jeweils einen weitgehend umlaufenden Außenrand 48, der durch eine Ausnehmung (Kerbe) für den elektrischen Kontakt 2 der betroffene Elektrode 16 unterbrochen ist. An den beiden Außenrändern 48 sind jeweils ein als Ringschnappverbindung dienender äußerer Wulst 50 oder eine als Ringschnappverbindung dienende innere Nut 52 gebildet.

Gemäß dieser Schrift ist also eine Ringschnappverbindung oder auch eine (nicht gezeigte) Clipsverbindung als einseitige Vorrichtung zu verstehen. Zwei derartige Vorrichtungen bilden zusammen eine Einspannung für die betroffene Elektrode 16.

In jedem Flansch 44 sind zwei außermittige Querbohrungen vorgesehen, die als Bügellöcher 54 dienen, und die mit Bezug zu Fig. 10 genauer erläutert werden.

Fig. 10 zeigt die Anordnung aus Fig. 9 im zusammengesetzten Zustand. Zusätzlich sind außen noch zwei weitere Elektroden 16 gezeigt. Es sind die drei Beispiele der Elektroden 16 aus den Figs. 2 bis 8 kombiniert, um die modulare Austauschbarkeit bzw. Kompatibilität der Elektroden 16 mit den Abstandhaltern 4, 6 zu zeigen.

Die mittlere Elektrode, von der nur der elektrische Kontakt 2 zu sehen ist, hat eine Einspannung. Die Einspannung ist von der inneren Nut 52 (erste Ringschnappverbindung) und dem darin eingeschnappten äußeren Wulst 50 (zweite Ringschnappverbindung) gebildet. Durch den weitgehend und fast geschlossenen Umlauf des Wulstes 50 und der Nut 52 am Außenrand 48 ist eine stabile Befestigung der Abstandhalter 4, 6 aneinander und eine dichte Einspannung der Elektrode erreicht.

Eine optionale Sicherung ist durch zwei metallische Bügel 56 realisiert, von denen in Figs. 9 und 10 jeweils nur ein Bügel 56 gezeigt ist. Die Bügel 56 haben jeweils zwei etwa parallel Schenkel 58, die in die Bügellöcher 54 zweier aneinander anliegende Flansche 44 eingesteckt werden. Wenn die beiden Schenkel 58 eines Bügels 56 gegeneinander gespannt sind, ist eine zweite Einspannung gegeben.

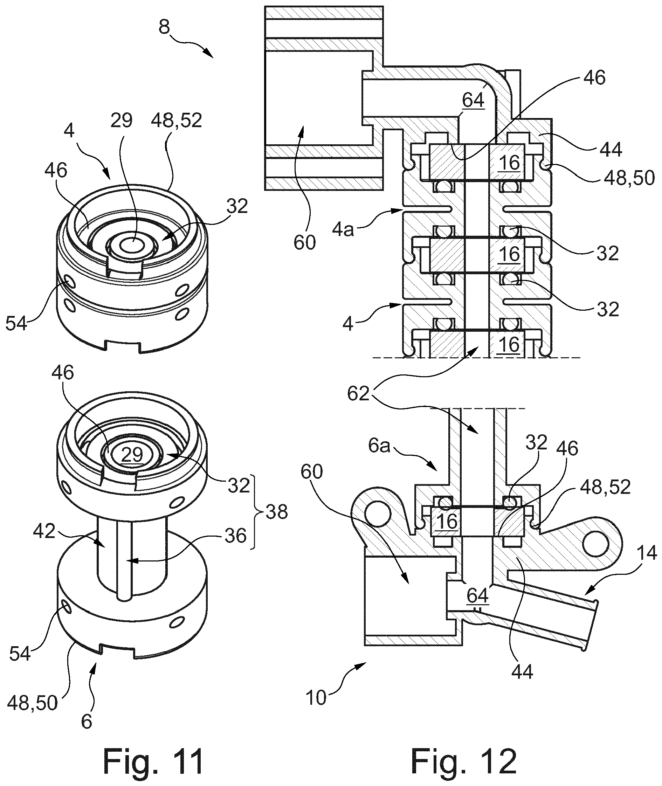

Fig. 11 zeigt eine lange und eine kurze Version eines zweiten Beispiels eines Abstandhalters 4, 6. Dabei entsprechen die Außenränder 48 mit den als Wulst 50 und als Nut 52 gebildeten Ringschnappverbindungen und den zusätzlichen Bügellöchern 54 denjenigen der Abstandhalter 4, 6 aus Figs. 9 und 10.

Wenn die Abstandhalter 4, 6 gemäß Fig. 11 mit den Elektroden 16 der Figs. 2 bis 8 kombiniert werden, dann entfallen deren Dichtringe 32 bzw. Dichtbauteile 38, denn es sind Dichtringe 32 an den Abstandhaltern 4, 6 vorgesehen. Es können also die mit Bezug zu den Figs. 2 bis 8 beschriebenen Aufbauten der Elektroden 16 aus elektrisch leitendem Bauteil und Hauptkörper 22 mit den dort genannten Herstellungsverfahren allerdings ohne Dichtringe 32 bzw. Dichtbauteile 38 in die Abstandhalter 4, 6 gemäß Fig. 11 eingespannt werden.

Bei den Abstandhaltern 4, 6 gemäß Fig. 11 ist an jeder Anlagefläche 46 ein Dichtring 32 vorgesehen. Die beiden Dichtringe 32 sind über zwei Verbindungselemente 36 miteinander einstückig verbunden, von denen in Fig. 11 nur ein Verbindungelement 36 des längeren Abstandhalters 6 zu erkennen ist. Die Verbindungselemente 36 erstrecken sich an der Außenseite des Halses 46. Damit ist ein formschlüssig befestigtes Dichtbauteil 38 am Abstandhalter 4, 6 gebildet.

Alle gezeigten Abstandhalter 4, 6 können im Kunststoff-Spritzguss-Verfahren aus biokompatiblem PPE+PS oder PEI oder PSU hergestellt werden. Bei den Abstandhaltern 4, 6 aus Fig. 11 ist das einstückige Dichtbauteil 38 aus Silikon in einem Schuss angespritzt.

Fig. 12 zeigt einen oberen und einen unteren Ausschnitt der Leitfähigkeitssonde 1 aus Fig. 1 mit den Abstandhaltern 4, 6 aus Fig. 11, also mit jeweiligem Dichtbauteil 38 mit zwei formschlüssig befestigten Dichtringen 32. Es sind mehrere Elektroden 16 entlang dem Messkanal aufgereiht, die keine eigenen Dichtelemente haben. Nur zwischen den beiden äußersten Elektroden 16 und dem jeweiligen Abschlussflansch 8, 10 muss noch ein extra Dichtring (nicht gezeigt) ergänzt werden.

Es ist zu erkennen, dass auch die beiden Abschlussflansche 8, 10 jeweils eine Ringschnappverbindung haben. Genauer gesagt weist der eine (in Figur 12 obere) Abschlussflansch 8 an dem Außenrand 48 seiner Anlagefläche 46 einen äußeren Wulst 50 auf, während der andere (in Figur 12 untere) Abschlussflansch 10 an dem Außenrand 48 seiner Anlagefläche 46 eine innere Nut 52 ausweist. Damit ist für alle Elektroden 16 also auch für die beiden äußersten Elektroden 16 jeweils eine individuelle Einspannung, die bei der Leitfähigkeitssonde 1 gemäß Fig. 12 alle von Ringschnappverbindungen, genauer gesagt von äußeren Wülsten 50 oder innere Nuten 52 gebildet sind.

Die beiden Abschlussflansche 8 ,10 verbinden jeweils einen Endabschnitt des aus den Durchgangsausnehmungen 18 der Elektroden 16 und den Durchgangskanälen der Abstandhalter 4, 6 gebildeten Messkanal 62 über eine abgewinkelten Kanal 64 mit dem Dialysegerät. An jedem abgewinkelten Kanal 64 ist ein Sensoraufnahme 60 gebildet. Dort kann ein Sensor z.B. für Temperatur, Druck und/oder Flussgeschwindigkeit eingesetzt werden.

### Bezugszeichenliste

- 1: Leitfähigkeitssonde
- 2: elektrischer Kontakt
- 4: kürzerer Abstandhalter
- 4a: äußerster kürzerer Abstandhalter
- 6: längerer Abstandhalter
- 6a: äußerster längerer Abstandhalter
- 8: Abschlussflansch
- 10: Abschlussflansch
- 12: Überwurfhülse
- 14: Schlauchtülle
- 16: Elektrode
- 18: Durchgangsausnehmung
- 20: Innenmantel
- 21: Stanz-Biege-Bauteil
- 22: Hauptkörper
- 24: Verbindungsabschnitt
- 26: Flansch
- 28: Zylinder
- 30: Stirnseite
- 32: Dichtring
- 34: Verbindungsloch
- 36: Verbindungselement
- 38: Dichtbauteil
- 39: Durchgangsloch
- 40: Kontaktstütze
- 42: Hals
- 44: Analgebereich / Flansch
- 46: Anlagefläche
- 48: Außenrand
- 50: äußerer Wulst
- 52: innere Nut
- 54: Bügelloch
- 56: Bügel
- 58: Schenkel
- 60: Sensoraufnahme
- 62: Messkanal
- 64: abgewinkelter Kanal

## Patentansprüche

1. Elektrode (16) mit einem Hauptkörper (22), der eine Durchgangsausnehmung (18) hat, die zwei zueinander parallele Stirnseiten (30) des Hauptkörpers (22) miteinander verbindet, wobei in der Durchgangsausnehmung (18) ein elektrisch leitfähiger Innenmantel (20) angeordnet ist, der von einer zu messender Dialysierflüssigkeit oder von deren Komponenten durchströmbar ist, wobei die Elektrode (16) weiterhin einen am Außenumfang des Hauptkörpers (22) angeordneten elektrischen Kontakt (2) hat, **dadurch gekennzeichnet, dass** der Innenmantel (20) und der elektrische Kontakt (2) gemeinsam aus einem ersten Material gebildet sind, und dass der Hauptkörper (22) ein von dem Innenmantel (20) und dem elektrischen Kontakt (2) getrenntes Trägerbauteil aus einem zweiten Material besteht.

2. Elektrode (16) nach Anspruch 1, **dadurch gekennzeichnet, dass** der elektrische Kontakt (2) ein Flachkontakt und/oder ein Steckkontakt ist.

3. Elektrode (16) nach einem der vorhergehenden Ansprüche, wobei an zumindest einer der Stirnseiten (30) ein Dichtring (32) angeordnet ist, der eine an der Stirnseite (30) gebildete Mündung der Durchgangsausnehmung (18) vollumfänglich umgibt, **dadurch gekennzeichnet, dass** der Dichtring (32) stoffschlüssig an der Stirnseite (30) befestigt ist.

4. Elektrode (16) nach Anspruch 1 oder 2, wobei an beiden Stirnseiten (30) jeweils ein Dichtring (32) angeordnet ist, der die jeweilige an der Stirnseite (30) gebildete Mündung der Durchgangsausnehmung (18) vollumfänglich umgibt, **dadurch gekennzeichnet, dass** die beiden Dichtringe (32) gemeinsam mit mindestens einem Verbindungselement (36) ein einstückiges Dichtbauteil (38) bilden, wobei sich das Verbindungselement (36) zwischen den beiden Dichtringen (32) durch ein als Durchgangsloch gebildetes Verbindungsloch (34) des Hauptkörpers (22) erstreckt.

5. Elektrode (16) nach einem der vorhergehenden Ansprüche, **dadurch gekennzeichnet, dass** der Hauptkörper (22) biokompatibles PPE und PS oder PEI oder PSU zumindest enthält.

6. Elektrode (16) nach einem der vorhergehenden Ansprüche, **dadurch gekennzeichnet, dass** der Innenmantel (20) und der elektrische Kontakt (2) Kupfer oder Messing oder Hartgold oder Graphit zumindest enthalten oder damit beschichtet sind.

7. Elektrode (16) nach einem der vorhergehenden Ansprüche, **dadurch gekennzeichnet, dass** der elektrische Kontakt (2) über einen Verbindungsabschnitt (24) mit dem Innenmantel (20) verbunden und elektrisch kontaktiert ist.

8. Elektrode (16) nach einem der vorhergehenden Ansprüche, **dadurch gekennzeichnet, dass** der Innenmantel (20) und der elektrische Kontakt (2) ein einstückiges Stanz-Biege-Bauteil (21) bilden, und dass der Hauptkörper (22) ein Kunststoff-Spritzguss-Teil ist, das um den Innenmantel (20) und um den Verbindungsabschnitt (24) herum gespritzt ist.

9. Elektrode (16) nach den Ansprüchen 7 und 8, **dadurch gekennzeichnet, dass** das Stanz-Biege-Bauteil (21) einen den Innenmantel (20) ausbildenden Zylinder (28) hat, an dem stirnseitig ein Flansch (26) gebildet ist, der an einer der Stirnseiten (20) angeordnet ist, und von dem sich der Verbindungsabschnitt (24) weg erstreckt.

10. Elektrode (16) nach einem der Ansprüche 1 bis 7, **dadurch gekennzeichnet, dass** der Hauptkörper (22) eine einstückig gebildete Kontaktstütze (40) umfasst, an der einseitig der elektrische Kontakt (2) angeordnet ist.

11. Elektrode (16) nach Anspruch 10, **dadurch gekennzeichnet, dass** der Innenmantel (20) und der elektrische Kontakt (2) per MID-Technik (molded interconnect device) oder mittels einer ähnlichen Beschichtungstechnik als Beschichtung auf den Hauptkörper (22) und auf dessen Kontaktstütze (40) aufgebracht sind/ist.

12. Elektrode (16) nach den Ansprüchen 7 und 11, wobei der Verbindungsabschnitt (24) auf eine der beiden Stirnseiten (30) des Hauptkörpers (22) aufgebracht ist.

13. Elektrode (16) nach einem der Ansprüche 1 bis 7, **dadurch gekennzeichnet, dass** der Innenmantel (20) und der elektrische Kontakt (2) und der Hauptköper (22) mittels eines additiven Verfahrens gebildet sind.

14. Elektrode (16) nach den Ansprüchen 7 und 13, wobei der Verbindungsabschnitt (24) im Innern des Hauptkörpers (22) angeordnet ist.
